(19) Europäisches Patentamt

European Patent Office

Office européen des brevets

(11) **EP 3 323 444 A1**

(12) **DEMANDE DE BREVET EUROPEEN**

(43) Date de publication:
**23.05.2018 Bulletin 2018/21**

(51) Int Cl.:
*A61M 1/36* (2006.01)        *B01D 43/00* (2006.01)
*B01D 21/28* (2006.01)       *B01J 19/10* (2006.01)
*C12M 1/00* (2006.01)

(21) Numéro de dépôt: **17202179.2**

(22) Date de dépôt: **16.11.2017**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**MA MD**

(30) Priorité: **17.11.2016 FR 1661161**

(71) Demandeur: **Commissariat à l'Energie Atomique
et aux Energies
Alternatives
75015 Paris (FR)**

(72) Inventeurs:
• **MINVIELLE, Zoé
  38000 GRENOBLE (FR)**
• **CWICKLINSKI, Grégory
  38000 GRENOBLE (FR)**

(74) Mandataire: **Brizio Delaporte, Allison
Cabinet Hautier
20, rue de la Liberté
06000 Nice (FR)**

(54) **PROCÉDÉ ET DISPOSITIF DE CONCENTRATION DE PARTICULES PAR ACOUSTOPHORÈSE**

(57)    L'invention concerne un procédé de concentration d'un milieu (1) contenant des particules micrométriques dispersées dans un fluide (3) par acoustophorèse caractérisé en ce qu'il comprend une circulation en continu et suivant un flux laminaire, du milieu (1) à concentrer successivement au travers de n zones (5) de concentration, une application, dans chaque zone (5) de concentration, d'un champ acoustique stationnaire de fréquence de résonnance propre sur le flux du milieu (1) à concentrer de sorte à agglomérer les particules sous forme d'une pluralité de bandes (2), et une récupération d'un premier sous-milieu comprenant les particules sous forme de bandes (2) à la sortie des n zones (5) de concentration, dans lequel la fréquence de résonnance du champ acoustique émis dans la nième zone (5a) de concentration est supérieure à la fréquence de résonnance du champ acoustique émis dans la nième + 1 zone (5b) de concentration de sorte à agglomérer successivement les particules sous forme de bandes (2) de plus en plus espacées.

Le domaine de l'invention concerne la concentration et la récupération de particules de taille micrométriques par acoustophorèse. Plus précisément, l'utilisation d'onde acoustique pour la concentration de particules de types micro-algues en aval d'un bioréacteur, ou d'autres particules non miscibles dans un fluide (3).

FIG. 2

**Description**

DOMAINE TECHNIQUE

La présente invention concerne un dispositif de concentration de particules par acoustophorèse et un procédé associé.

Le domaine de l'invention concerne la concentration et la séparation de particules de taille micrométrique par acoustophorèse. Plus précisément, l'invention concerne l'utilisation d'ondes acoustiques pour la concentration de particules de types micro-algues préalablement cultivées dans un bioréacteur, ou d'autres particules non miscibles dans un fluide.

ETAT DE LA TECHNIQUE

La méthode de concentration par acoustophorèse consiste en l'application d'un champ acoustique bi ou tridimensionnel. Ce champ est généré dans le fluide grâce à des céramiques piézoélectriques, appelées émetteur ou transducteur, et permet de mettre en mouvement des particules dans un milieu fluide sous l'effet d'une force acoustique. Le principe de concentration de particules par acoustophorèse est décrit notamment dans Optofluidics incorporating actively controlled micro- and nano-particles de Kayani et al. - BIOMICROFLUIDICS 6, 031501 (2012).

Pour générer un champ acoustique en deux dimensions, une plaque piézoélectrique émetteur placée sur la paroi latérale d'une cellule contenant le fluide est excitée par un courant électrique, ce qui engendre la propagation d'une onde plane dans le fluide. Cette onde est réfléchie par une deuxième plaque, dite réflecteur, placée parallèlement à la première sur la paroi opposée. La superposition des deux ondes émise et réfléchie forme une onde stationnaire. Un champ de pression bidimensionnel est donc formé dans le fluide et les particules sont concentrées dans des endroits spécifiques, noeuds ou ventres de pression, selon le signe de leur contraste acoustique, sous l'effet des forces acoustiques.

Le contraste acoustique est, selon son signe, responsable des positions des particules. Il dépend des propriétés du milieu et de la particule. Si le contraste acoustique est négatif : les particules sont attirées vers les ventres de pression, sinon elles sont attirées vers les noeuds.

L'acoustophorèse est notamment utilisée dans le milieu médical pour le tri de cellules sanguines. Le document WO2010/036667 décrit un appareil et un procédé pour la séparation de particules suspendues dans un liquide. Ce dispositif fonctionne à des fréquences de 1 ou plusieurs MHz pour des particules micrométriques de sorte à déplacer les particules à séparer au centre du flux à traiter. Le canal est dimensionné pour permettre la formation d'une seule bande de concentration au centre du canal.

Les particules, soumises au champ acoustique, se concentrent aux noeuds (ou ventres) de pression distants d'une demi-longueur d'onde. Ainsi dans de l'eau et pour une fréquence de 1 MHz, cette distance vaut 750 $\mu$m environ, ce qui impose des conduits de séparation dans le cas d'un dispositif continu de quelques centaines de micromètre seulement.

Pour des applications type prélèvement sanguin, les débits traités sont faibles et compatibles avec de telles dimensions.

En revanche, pour d'autres applications nécessitant de plus gros volumes à traiter, par exemple type micro algues, dont l'objectif est d'aller vers la production de produits de grande consommation comme les biocarburants par exemple, ces échelles micrométriques peuvent être problématiques pour la mise en oeuvre industrielle.

Il existe le besoin de proposer un dispositif et un procédé pour la concentration et la séparation de particules de tailles micrométriques adaptés à une mise en oeuvre industrielle avec des volumes de fluide pouvant être traités importants.

EXPOSE DE L'INVENTION

La présente invention propose à cet effet un procédé de concentration d'un milieu contenant des particules micrométriques dans un fluide, par acoustophorèse, comprenant une étape de circulation en continu du milieu à concentrer suivant un flux laminaire dans n zones dites de concentration successives, une étape d'application, sur le milieu circulant, dans chaque zone de concentration, d'un champ acoustique stationnaire de fréquence de résonance propre de sorte à agglomérer les particules sous forme de bandes, une étape de récupération d'un premier sous milieu comprenant les bandes de particules agglomérées à la sortie des n zones de concentration. La fréquence de résonance du champ acoustique émis dans la nième zone de concentration est supérieure à la fréquence de résonance du champ acoustique émis dans la nième + 1 zone de concentration de sorte à agglomérer successivement les particules, pour augmenter leur taille caractéristique, sous forme de bandes de plus en plus espacées .

Avantageusement, les bandes sont plus larges et/ou plus espacées dans la n[ième]+1 zone de concentration que dans la n$^{ième}$ zone de concentration.

Avantageusement, le nombre n de zones de concentration est supérieur ou égal à 2.

Par cette disposition de l'invention, il est possible de concentrer et séparer des particules micrométriques avec des fréquences de résonnance de plus en plus faibles et des moyens de récupération de plus en plus larges. Le procédé selon l'invention utilise l'agglomération des particules micrométriques pour notamment augmenter la taille caractéristique des particules à séparer permettant d'appliquer une force acoustique de plus en plus basse. L'agglomération des particules entraine la formation de bande de plus en plus espacées et/ou de plus en plus larges facilitant ainsi la séparation et la récupération des particules. L'invention permet de réduire les contraintes sur la fabrication des moyens de récupération. Cette augmentation des dimensions notamment des moyens de récupération contribue à permettre l'augmentation du débit du flux à traiter et donc l'augmentation des volumes pouvant être traités. Cela est particulièrement avantageux en ce que les moyens de récupération selon l'invention permettent une récupération d'un premier sous milieu de sorte à concentrer les particules et non pas une séparation grossière.

De manière avantageuse, la fréquence de résonnance d'un nième champ acoustique émis dans une nième zone de concentration est supérieure d'un ordre de grandeur à une fréquence de résonnance d'un nième +1 champ acoustique émis dans une nième+1 zone de concentration.

Préférentiellement, le présent procédé permet la concentration de particules dans un milieu circulant avec un débit compris entre 1 et 200 L/h.

Suivant un autre aspect, l'invention concerne un dispositif de concentration d'un milieu circulant en continu contenant des particules micrométriques dans un fluide, par acoustophorèse, comprenant suivant le sens de circulation du milieu: une section d'entrée recevant le milieu à concentrer et configurée pour générer un flux laminaire continu du milieu, n zones de concentration successives, chaque zone de concentration comprenant une source ultrasonore générant un champ acoustique stationnaire de fréquence de résonnance propre, la fréquence de résonnance du champ acoustique dans une zone n étant supérieure à la fréquence de résonnance du champ acoustique dans une zone n+1 de sorte à agglomérer successivement les particules, pour augmenter leur taille caractéristique, sous forme de bandes de plus en plus espacées, et une section de récupération comprenant des moyens de récupération configurés pour récupérer un premier sous-milieu comprenant les particules agglomérées sous forme de bandes.

Avantageusement, les bandes sont plus larges et/ou plus espacées dans la n$^{ième}$+1 zone de concentration que dans la n$^{ième}$ zone de concentration.

Préférentiellement, le moyen de récupération est un peigne muni d'une pluralité de dents, chaque dent comprend un canal longitudinal destiné à séparer une bande de particules agglomérées, les dents étant espacées par un espace inter-dents au travers desquels circule un deuxième sous milieu, correspondant au fluide entre les bandes, il contient des particules à faible concentration, c'est un milieu dilué. Le peigne permet un prélèvement des bandes de particules agglomérées en limitant la quantité de milieu récupérée. Le peigne permet une concentration des particules.

## BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les figures d'accompagnement suivantes dans lesquelles :

Figure 1 : Vue en perspective d'un dispositif de l'invention
Figure 2 : Vue du dessus d'un moyen de récupération du premier sous milieu selon un mode de réalisation préférée de l'invention.

## EXPOSE DETAILLE DE MODES DE REALISATION PARTICULIERS

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention concerne un procédé de concentration d'un milieu contenant des particules micrométriques dispersées dans un fluide, par acoustophorèse, caractérisé en ce qu'il comprend : une circulation en continu et suivant un flux laminaire, du milieu à concentrer successivement au travers de n zones de concentration, une application, dans chaque zone de concentration, d'un champ acoustique stationnaire de fréquence de résonnance propre sur le flux du milieu à concentrer de sorte à agglomérer les particules sous forme d'une pluralité de bandes, et une récupération d'un premier sous-milieu comprenant les particules sous forme de bandes à la sortie des n zones de concentration, dans lequel la fréquence de résonnance du champ acoustique émis dans la nième zone de concentration est supérieure à la fréquence de résonnance du champ acoustique émis dans la nième + 1 zone de concentration de sorte à agglomérer successivement les particules sous forme de bandes de plus en plus espacées.

Avantageusement, suivant des variantes préférées mais non limitatives, l'invention est telle que :

Avantageusement, le procédé comprend une étape de récupération d'un premier sous milieu comprenant des bandes de particules à la sortie des n zones de concentration.

Avantageusement, les micro-algues sont en suspension dans le fluide.

Avantageusement, les n zones de concentration présentent une hauteur équivalente à au moins une demi-longueur d'onde du champ acoustique de plus grande fréquence.

Avantageusement, la circulation est paramétrée de sorte à ce que le milieu circule suivant un flux ayant un débit compris entre 1 et 200 L/h.

Avantageusement, la fréquence de résonnance du champ acoustique émis dans la nième zone de concentration est supérieure d'un ordre de grandeur à la fréquence de résonnance du champ acoustique émis dans la nième+1 zone de concentration.

Avantageusement, au moins un champ acoustique est orthogonal à la direction de circulation du milieu.

Avantageusement, les particules sont des micro-algues.

Avantageusement, les particules ont une taille inférieure à $100\mu m$, préférentiellement inférieure ou égale à $80\mu m$, plus préférentiellement comprise entre $5\mu m$ inclus et $80\mu m$ inclus.

Avantageusement, la fréquence de résonnance du champ acoustique émis dans la nième zone de concentration est comprise entre 1 et 10 MHz, de préférence égale à 2MHz, et la fréquence de résonnance du champ acoustique émis dans la nième+ 1 zone de concentration est comprise entre 150 et 400 kHz, de préférence égale à 250kHz.

Avantageusement, les zones de concentration sont configurées pour que les particules sous forme de bandes soient de plus en plus larges.

Suivant un autre aspect, l'invention concerne un dispositif un dispositif destiné à la concentration d'un milieu, circulant en continu, contenant des particules micrométriques dispersées dans un fluide, par acoustophorèse, caractérisé en ce qu'il comprend suivant un sens de circulation du milieu: une section d'entrée destinée à recevoir le milieu à concentrer et configurée pour générer un flux laminaire continu dudit milieu, n zones de concentration successives, chaque zone de concentration comprenant une source ultrasonore configurée pour générer un champ acoustique stationnaire de fréquence de résonnance propre, la fréquence de résonnance d'une zone n étant supérieure à la fréquence de résonnance d'une zone n+1 de sorte à agglomérer successivement les particules sous forme de bandes de plus en plus espacées, une section de récupération comprenant un moyen de récupération configuré pour récupérer un premier sous-milieu comprenant les particules agglomérées sous forme de bandes.

Avantageusement, suivant des variantes préférées mais non limitatives, l'invention est telle que :

- chaque source acoustique des zones de concentration comprend une première plaque piézoélectrique émettrice placée sur une paroi latérale d'une zone de concentration et une deuxième plaque piézoélectrique réflectrice placée parallèle à la première sur une paroi opposée de ladite zone de concentration de sorte à générer une onde acoustique stationnaire orthogonale au sens de circulation du milieu.
- la largeur des n zones de concentrations est configurée pour permettre la formation de plusieurs bandes de particules agglomérées parallèles les unes aux autres avec largeur = nombre de bandes x ½ longueur d'onde.
- la largeur des n zones de concentration est identique de sorte que les bandes soient de plus en plus larges.
- la longueur des n zones de concentration est configurée pour que le temps de séjour soit supérieur au temps de migration des particules.
- les n zones de concentration successives sont directement connectées les unes à la suite des aux autres.
- La section de récupération comprend un peigne muni d'une pluralité de dents, chaque dent comprenant un canal longitudinal destiné à récupérer une bande de particules, les dents étant espacées par un espace inter-dents au travers desquels circule un deuxième sous-milieu correspondant au milieu entre les bandes.
- le peigne est agencé de sorte que la direction longitudinale des canaux soit parallèle à la direction longitudinale des bandes de particules.
- le nombre de dents est égal au nombre de bandes dans la zone de concentration précédant directement la section de récupération.

Avantageusement, la largeur des zones de concentration est identique.

Eventuellement, les options suivantes sont aussi possibles :

Suivant un autre aspect, l'invention concerne un procédé de concentration d'un milieu contenant des particules micrométriques dispersées dans un fluide, par acoustophorèse, comprenant une étape de circulation en continu du milieu à concentrer suivant un flux laminaire dans n zones de concentrations successives, une étape d'application, sur le milieu à traiter circulant, d'une fréquence de résonnance stationnaire décroissante le long du sens de circulation du milieu de sorte à agglomérer les particules sous forme de bandes de plus en plus espacées, une étape de récupération d'un premier sous milieu comprenant des bandes de particules à la sortie des n zones de concentration.

Suivant un autre aspect l'invention concerne un procédé de concentration d'un milieu contenant des particules micrométriques dispersées dans un fluide, par acoustophorèse, dans lequel le premier sous milieu récupéré par le moyen de récupération à la sortie des n zones de concentration est remis en circulation en continu suivant un flux laminaire dans n zones de concentrations successives, puis est soumis dans chaque zone de concentration à un champ acoustique de fréquence de résonnance propre de sorte à agglomérer les particules sous forme de bandes, la fréquence de résonnance du champ acoustique émis dans la nième zone de concentration étant supérieure à la fréquence de résonnance du champ acoustique émis dans la nième + 1 zone de concentration de sorte à agglomérer successivement les particules sous forme de bandes de plus en plus espacées, un premier sous-milieu comprenant les bandes de particules est séparé d'un deuxième sous-milieu comprenant le fluide à la sortie des n zones de concentration.

Avantageusement, le dispositif comprend des moyens de refroidissement comprenant un fluide de refroidissement et un circuit de refroidissement configuré pour permettre la circulation du fluide de refroidissement, préférentiellement à contre-courant du milieu à concentrer, autour des zones de concentration.

Avantageusement, le circuit de refroidissement est agencé sur des parois inférieure et/ou supérieure des zones de concentration.

Suivant un autre aspect de l'invention, l'invention concerne un système d'au moins un premier et un deuxième dispositif, tel que décrit ci-dessus, en série, le deuxième dispositif étant alimenté par les bandes de particules récupérées par le moyen de récupération du premier dispositif.

Avantageusement, selon l'invention les particules sont de taille inférieure à $100\,\mu m$, préférentiellement inférieure à $80\,\mu m$, plus précisément comprise, en incluant, entre $5\,\mu m$ inclus et $80\,\mu m$ inclus. Les particules sont ainsi dénommées particules micrométriques ou bien microparticules.

Les particules du milieu 1 sont avantageusement homogènes en termes de taille.

Les particules à concentrer sont contenues dans un fluide 3 formant ainsi un milieu 1. Les particules sont dispersées dans le fluide. Le milieu comprend une phase dispersée, les particules, dans un milieu de dispersion, le fluide. Les particules peuvent être des particules solides ou des gouttelettes non miscibles dans un fluide 3 donné par exemple émulsion huile/eau ou fluide organique/phase aqueuse. Le fluide 3 peut être un liquide mais aussi un gaz.

L'invention trouvera son application plus particulièrement pour la séparation d'algues en sortie de bioréacteur. Les algues sont des algues micrométriques par exemple phaeodactylum, neochloris, crypthecodinium, asterionella, spirulina, chlorella vulgaris, chlamydomonas...Les microalgues sont en suspension dans le fluide 3.

Selon l'invention, le milieu 1 circule dans le dispositif en continu de sorte à être traiter par le procédé selon l'invention en continu. A cet effet, le dispositif comprend au moins une pompe d'alimentation mettant en circulation le milieu 1 à traiter dans le dispositif.

Le milieu 1 circule ou s'écoule dans le dispositif suivant un sens ou une direction de circulation ou d'écoulement.

Le dispositif est configuré pour traiter des débits du milieu 1 compris entre 1 et 200 L/h plus précisément de l'ordre de 90L/h. De tels débits permettent une utilisation du dispositif à des échelles industrielles tel que par exemple pour la fabrication de biocarburant.

Le dispositif selon l'invention comprend n zones 5 successives dites de concentration. Le flux de milieu 1 à traiter comprenant les particules à concentrer dans un fluide 3 circule dans le dispositif et plus précisément successivement au travers des n zones 5 de concentration. Le nombre n de zone de concentration est supérieur ou égal à 2. Plus le nombre de zones augmente plus la dépense énergétique est importante, préférentiellement le nombre de zone est inférieur ou égal à 6. L'augmentation du nombre de zone de concentration permet notamment de concentrer et séparer des particules de plus faible taille.

Selon l'invention, chaque zone de concentration comprend une source ultrasonore de fréquence de résonnance propre générant un champ acoustique. On entend par fréquence de résonnance propre que la fréquence de résonnance est propre à chaque zone 5, c'est-à-dire que la fréquence de résonnance est caractéristique de ladite zone 5, elle est spécifiquement appliquée dans ladite zone 5.

Les zones 5 se succèdent sans séparation physique. Les zones se distinguent les unes des autres par le champ acoustique émis.

Les n zones 5 s'étendant suivant le sens de circulation 12 du milieu 1 dans le dispositif. La zone n précède la zone n+1 suivant le sens de circulation 12 du milieu 1. Préférentiellement, les zones 5 de concentration sont directement connectées les unes à la suite des autres. Les zones 5 se succèdent continûment deux à deux. De cette manière, le milieu 1 est avantageusement constamment soumis à un champ acoustique dans les zones 5 de concentration.

La fréquence de résonnance du champ acoustique émis dans la zone n est supérieure à la fréquence de résonnance du champ acoustique émis dans la zone n+1. Le milieu 1 est soumis à une fréquence de résonnance décroissante au cours de sa circulation au travers des zones 5 de concentration. Avantageusement, la décroissance de la fréquence de résonnance est réalisée de manière discontinue, c'est-à-dire que la décroissance se fait par palier correspondant avantageusement à chaque zone de concentration, c'est-à-dire que dans chaque zone de concentration la fréquence de résonnance est stable mais différente de la fréquence de résonnance de la zone de concentration adjacente, c'est-à-dire de la précédente et de celle de la suivante.

Les sources ultrasonores émettent simultanément une fréquence de résonnance propre à chaque zone 5.

Avantageusement, la fréquence de résonnance de la n zone de concentration est supérieure d'un ordre de grandeur à la fréquence de résonnance de la nième+1 zone de concentration. Suivant une préférence, la fréquence de résonnance de la n zone de concentration est comprise entre 1 et 10MHz, par exemple préféré 2MHz et la fréquence de résonnance de la nième+1 zone de concentration est comprise entre 150 et 400kHz, par exemple préféré 250kHz.

Cette utilisation de fréquence acoustique différente et décroissante permet d'appliquer une force acoustique suffisante pour agglomérer les microparticules, préférentiellement sous forme d'une pluralité de bandes 2. Les bandes 2 sont des agglomérats de microparticules, on entend par agglomérat un amas de microparticules donnant une apparence unifiée.

Les particules soumises dans le fluide 3 à un champ acoustique se concentrent aux noeuds ou aux ventres de pression de l'onde de pression acoustique.

Les bandes sont des agglomérats de particules de forme étroite et allongée. Les bandes se forment sur toute la hauteur de la zone de concentration 5.

Au début de l'écoulement du milieu 1, plus précisément dans la première zone 5a de concentration, les particules sont de faible taille caractéristique correspondant sensiblement à leur taille réelle. Le milieu 1 est soumis à une première fréquence de résonnance élevée de sorte à créer une force acoustique suffisante pour concentrer les particules aux noeuds ou aux ventres de l'onde acoustique. La force acoustique est fonction de la taille des particules et de la fréquence, plus précisément, la force acoustique est proportionnelle au rayon au cube des particules.

On entend par taille caractéristique des particules, la taille apparente pour le champ acoustique. La taille caractéristique est un ordre de grandeur. Ainsi, pour les particules micrométriques en entrée du dispositif on pourra prendre comme taille caractéristique le rayon ou le diamètre ou tout autre distance de cet ordre de grandeur selon la convention choisie et pour les particules agglomérées on pourra prendre comme taille caractéristique la largeur d'une bande ou bien sa hauteur ou tout autre distance de cet ordre de grandeur selon la convention choisie.

A l'issu de cette première zone 5a, les particules sont agglomérées et présentent une taille caractéristique plus élevée qu'à l'entrée de cette zone, plus précisément d'un facteur 10 par rapport à leur diamètre initial, permettant l'application dans une deuxième zone 5b d'une fréquence de résonnance plus faible pour exercer une force acoustique suffisante pour concentrer les particules aux noeuds ou aux ventres de l'onde acoustique.

Lorsque la largeur I des zones de concentration 5 est identique, la largeur des bandes 2 augmentent au cours de l'écoulement du milieu 1 dans le dispositif. Les bandes de particules sont avantageusement de plus en plus larges facilitant ainsi la récupération des particules. Les bandes sont de plus en plus larges d'une zone 5a à une autre 5b.

Les noeuds ou ventres de l'onde acoustique sont distants d'une demi-longueur d'onde avec $\lambda = c / f$ dans lequel A est la longueur d'onde de l'onde acoustique, « c » est la vitesse de l'onde dans le fluide et f est la fréquence de résonnance. Par exemple, dans de l'eau et pour une fréquence de 1 MHz, cette distance vaut 750 $\mu$m environ.

Ainsi, en diminuant la fréquence, la distance augmente jusqu'à 5 mm pour une fréquence de 150kHz.

Au cours de l'écoulement, les bandes sont de plus en plus espacées quelques soit les dimensions des zones de concentrations.

Si la largeur de chaque zone 5 est identique, l'espacement 10 entre les bandes 2 augmente tandis que le nombre de bande diminue au cours de l'écoulement du milieu 1 dans le dispositif et la taille caractéristique des particules augmente, les bandes sont de plus en plus larges.

Le milieu 1 circulant est séparé en un premier sous-milieu contenant une première concentration de particules et un deuxième sous-milieu contenant une deuxième concentration de particules. La concentration en particules du premier sous milieu étant plus élevée que la concentration en particules du deuxième sous-milieu. Le premier sous-milieu correspond aux bandes de particules, il contient des particules à forte concentration dans le fluide. Le deuxième sous-milieu correspond au fluide entre les bandes, il contient des particules à faible concentration, avantageusement nulle, dans le fluide, aussi dénommé fluide ou milieu dilué.

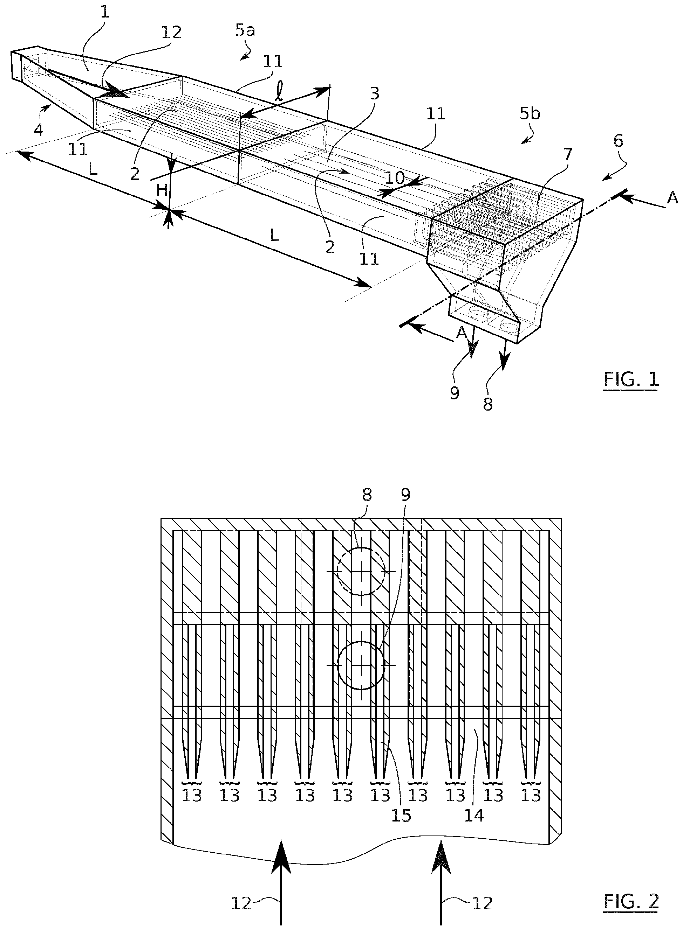

Suivant la figure 1, le dispositif selon l'invention comprend un canal de circulation du milieu 1. Le canal est de forme parallélépipédique, avantageusement rectangulaire ou carré. Le canal comprend une paroi inférieure formant le fond, deux parois latérales 11 et avantageusement une paroi supérieure. Le canal est orienté de sorte que la circulation du milieu 1 soit horizontale. Il n'y a avantageusement pas de pente. Avantageusement, la circulation du milieu 1 est due l'action d'une pompe propulsant le milieu 1 dans le dispositif. Les zones 5 de concentration sont formées le long du canal. Le canal d'écoulement au niveau des zones 5 de concentration présente une largeur (I) configurée pour permettre la formation d'une pluralité de bandes 2 de concentration parallèles entre elles. Les bandes 2 sont avantageusement parallèles à une direction principale de circulation du milieu 1. La largeur I du canal est avantageusement proportionnelle à la longueur d'onde. La largeur I est égale au nombre de bandes 2 souhaitées par la ½ longueur d'onde. Ainsi, pour une largeur I fixe, plus la longueur d'onde augmente quand la fréquence baisse, moins le nombre de bandes 2 est élevé. La largeur I du canal des différentes zones 5 de concentration reste avantageusement identique et la fréquence de résonnance du champ acoustique baisse (la longueur d'onde augmente) ce qui conduit à une diminution du nombre de bandes 2. Les bandes 2 de concentration sont avantageusement plus larges. La concentration en particules du premier sous milieu augmente tandis que la concentration en particules du deuxième sous milieu diminue.

La hauteur du canal de circulation du milieu 1 au niveau des zones 5 de concentration est fonction de la longueur d'onde du champ acoustique de plus grande fréquence appliquée dans les zones 5 de concentration, avantageusement elle est inférieure ou égale à 5, préférentiellement elle est équivalente à une demi-longueur d'onde du champ acoustique de plus grande fréquence appliquée dans les zones 5 de concentration. Cette hauteur correspond à la hauteur du milieu 1 circulant dans lesdites zones 5 de concentration.

La longueur L de chaque zone de concentration est configurée pour que le temps de séjour soit supérieur au temps de migration des particules. Le temps de séjour est égal au rapport de la longueur L de la zone de concentration par la vitesse de circulation. Le temps de migration est lui fonction de la fréquence de résonnance et de la taille caractéristique des particules. Le temps de migration résulte d'un bilan des forces appliquées à la particule à savoir la traînée, la gravité et la force acoustique. Cette dernière dépend entre autres du rayon au cube de la particule, ou de la taille caractéristique qui sera considérée pour les particules, et de la fréquence. Ce bilan des forces permet de déduire la vitesse de déplacement de la particule et donc le temps de migration jusqu'au noeud (ou ventre) de pression selon sa position initiale.

Le dispositif comprend des sources ultrasonores. Les sources ultrasonores sont agencées dans chacune des zones 5 de concentration. Chaque source ultrasonore comprend un émetteur positionné sur une première face orthogonale au sens d'écoulement du milieu 1, c'est-à-dire une paroi latérale 11, et avantageusement un réflecteur destiné à réfléchir l'onde acoustique positionné sur une face opposée à la première face, c'est-à-dire une paroi latérale 11. L'émetteur est par exemple une plaque piézoélectrique. Le réflecteur est une plaque préférentiellement non émettrice.

L'onde ultrasonore générée par cette source ultrasonore est une onde stationnaire ce qui permet de déplacer puis maintenir les particules à concentrer au niveau des noeuds ou ventres de pression.

Avantageusement, le dispositif comprend une section d'entrée 4 configurée pour générer un flux laminaire dans les zones 5 de concentration. La section d'entrée 4 précède, les zones 5 de concentration suivant le sens de circulation 12 du milieu 1. Selon l'invention, le milieu 1 s'écoule dans les zones 5 de concentration suivant un flux laminaire de sorte à éviter des recirculations du milieu 1 qui viendraient remélanger les bandes 2 de concentrations. La force acoustique ne permet pas de compenser les turbulences du milieu 1. Préférentiellement, la section d'entrée 4 est un canal divergent débouchant dans les zones 5 de concentration.

Avantageusement, le dispositif comprend une section de récupération 6 comprenant un moyen de récupération des particules sous formes de bandes 2, plus précisément du premier sous milieu. Le moyen de récupération est un peigne 7 muni d'une pluralité de dents 13. Chaque dent 13 comprend un canal longitudinal, aussi dénommé espace intra-dent 15, destiné à récupérer principalement le premier sous-milieu c'est à dire les particules concentrées sous forme de bandes 2 avec un minimum de fluide 3, c'est-à-dire de deuxième sous-milieu de sorte à ne pas diluer les bandes 2 de concentration. Les dents 13 du peigne 7 sont orientées parallèlement aux bandes 2 de concentrations. Les dents 13 du peigne 7 sont avantageusement en même nombre que le nombre de bandes 2 de concentration dans la zone de concentration précédant directement la section de récupération 6. Les dents 13 du peigne 7 sont préférentiellement disposées en regard desdites bandes 2 de concentration de sorte à ce que les particules pénètrent directement dans un canal longitudinal d'une dent 13.

La largeur du canal d'une dent 13, aussi dénommée espace intra-dent 15, est définie en fonction du facteur de concentration requis en sortie.

L'espace intra-dent 15 (là où est prélevé le 1$^{er}$ sous-milieu) du peigne 7 est défini comme ci-après.

Le dispositif selon l'invention permet un facteur de concentration des particules comprise entre 2 et 20, plus précisément entre 3 et 6, plus particulièrement 3,8.

Le facteur de concentration étant Cf/Ci avec

$$Ci = \frac{m_{\text{particules}}}{V_{\text{dispositif}}}$$

$$Cf = \frac{m_{\text{particules}}}{V_{\text{(dent peigne)}}}$$

Ci et Cf (kg/m$^3$eau) sont respectivement les concentrations en particules initiale (milieu à traiter) et finale (premier sous milieu c'est-à-dire bandes 2 de concentration).

V$_{\text{dispositif}}$ est le volume des zones 5 de concentration.

V$_{\text{(dent peigne)}}$ est le volume du 1$^{er}$ sous-milieu prélevé par l'espace intra-dent 15 du peigne 7.

Le volume des zones 5 de concentration correspond au produit de la largeur I par la hauteur h par la longueur L de la zone de concentration 5.

Le volume prélevé correspond au produit de la longueur L du dispositif plus précisément des zones 5 de concentration par la hauteur h des zones 5 de concentration par l'espace intra-dent 15 du peigne 7 par le nombre de dents 13, ce dernier étant égal au nombre de bandes 2 de concentration. Le nombre de bande étant égal à la largeur l des zones 5 de concentration du dispositif divisée par une demi-longueur d'onde.

Après simplifications, le facteur de concentration s'écrit :

$$\frac{Cf}{Ci} = \frac{(\lambda/2)}{\text{espace intra} - \text{dent}}$$

Selon l'invention, l'espace intra-dent 15 du peigne 7 est compris entre 0,400 et 1 mm.

Plus l'espace intra-dent 15 sera réduit, plus le premier sous milieu récupéré sera concentré.

Le moyen de récupération comprend en outre deux conduits d'évacuation, l'un 8 pour la sortie du premier sous milieu, c'est-à-dire des particules, connecté aux canaux longitudinaux des dents 13 et l'autre 9 pour la sortie du deuxième sous milieu, c'est-à-dire du fluide dilué, connecté à l'espace inter-dents 14 du peigne 7.

Le premier sous milieu récupéré dans le conduit de sortie 8 peut être à nouveau traité dans un dispositif tel que décrit ci-dessus. Suivant ce mode de réalisation, le deuxième dispositif est agencé en série à la suite du premier dispositif. Dans ce cas, l'invention concerne un système de dispositifs agencés en série. Le deuxième dispositif traitant avantageusement le premier sous -milieu du premier dispositif.

Suivant un mode de réalisation, le dispositif comprend des moyens de refroidissement comprenant un fluide de refroidissement et un circuit de refroidissement configuré pour permettre la circulation du fluide de refroidissement, préférentiellement à contre-courant du milieu à concentrer, autour des zones de concentration. Ce dispositif de refroidissement est particulièrement utile dans le cas où les émetteurs seraient puissants et/ou le débit à traiter faible et que le milieu s'échaufferait lors du passage dans les zones de concentration. Un échauffement pourrait être particulièrement néfaste suivant la nature des particules et notamment pour les micro-algues. Le refroidissement permet aussi de stabiliser la température du milieu 1 circulant et donc le champ acoustique. En effet, la vitesse du son dans le milieu fluide dépend de la température donc quand la température varie, la vitesse du son varie et donc la longueur d'onde varie. Par conséquent le nombre de bandes varie ainsi que leur position (égale à ½ longueur d'onde). Du coup les dents du peigne pourraient se retrouver décalées par rapport aux bandes de concentration, ce qui est gênant pour la récupération.

Le fluide de refroidissement est par exemple préféré de l'eau.

Avantageusement, le circuit de refroidissement est agencé sur des parois inférieure et/ou supérieure des zones de concentration.

EXEMPLE

Un milieu 1 à traiter comprenant des algues de taille caractéristique de l'ordre de 80$\mu$m en suspension dans un fluide 3 équivalent à de l'eau. Le milieu 1 traverse le dispositif en continu suivant un flux laminaire avantageusement générer par la section d'entrée 4 du dispositif.

Le dispositif selon l'invention comprend deux zones 5 de concentration successives et directement reliées.

La première zone de concentration, correspondant à la première zone suivant le sens de circulation 12 du milieu 1, comprend une première source ultrasonore émettant un premier champ acoustique avec une fréquence de résonnance de 2 MHz. Les micro algues sont concentrées en bandes 2 espacées de 30$\mu$m.

Les particules agglomérées dans le fluide 3 circulent ensuite dans la deuxième zone de concentration, correspondant à la deuxième zone suivant le sens de circulation 12 du milieu 1, comprend une deuxième source ultrasonore émettant un deuxième champ acoustique avec une fréquence de résonnance de 250 kHz. Les particules pénétrant dans cette deuxième zone ont une taille caractéristique supérieure à leur taille caractéristique en entrée de la première zone puisque qu'elles sont agglomérées. Leur taille caractéristique correspond ici au moins à la largeur des bandes 2 de concentration, notamment la largeur exposée à l'onde acoustique dans la première zone de concentration. Les micro algues sont concentrées en bandes 2 espacées de 3mm.

Suivant cet exemple, la largeur des zones 5 de concentration est définie pour avoir 15 bandes 2 dans la deuxième zone de concentration à 250 kHz soit une largeur de 50mm. La hauteur h des zones 5 de concentration est par exemple définie suivant la plus petite longueur d'onde soit une hauteur h de 3mm. La longueur des zones 5 de concentration est de 50 mm par exemple.

Le peigne 7 du moyen de récupération comprend 15 dents 13. L'espacement entre les bandes étant de 3mm, pour avoir un facteur de concentration de 3,8, l'espace intra-dent 15 est de 800$\mu$m.

Avec un dispositif de ce type, le débit du milieu 1 pouvant être traité est de 90L/h suivant le calcul ci-dessous :

On travaille en régime laminaire, par exemple avec un nombre de Reynolds de 1000. Celui-ci s'écrit :

$$Re = \frac{\rho \cdot u \cdot d}{\mu}$$

p et $\mu$ sont la masse volumique et la viscosité du milieu 1 à traiter (que l'on peut assimiler à de l'eau, soit 1000 kg-m-3 et 1.10-3 Pa·s).

d (m) est le diamètre hydraulique du dispositif. Il s'écrit :

$$d = \frac{4 \cdot S}{P}$$

S est la section de passage (m2) et P le périmètre mouillé (m), ils valent donc :

S= largeur x hauteur de la zone de concentration

S= 50x3

S= 150 mm2

P= 2 x (largeur + hauteur de la zone de concentration)

P= 2x(50+3)

P= 106 mm

A partir de l'expression du Reynolds, on obtient la vitesse de l'écoulement, u (m/s) et donc le débit maximal pouvant être traité soit environ 90 L/h.

REFERENCES

1. Milieu
2. Bande
3. Fluide
4. Section d'entrée
5. Zone de concentration

    5a. première zone
    5b. deuxième zone

6. Section de récupération
7. Peigne
8. Conduit de sortie du premier sous-milieu
9. Conduit de sortie du deuxième sous-milieu
10. Espacement entre les bandes
11. Parois latérales
12. Sens de circulation du milieu
13. Dent
14. Espace inter-dents
15. Espace intra-dent

l. Largeur des zones de concentration
L. Longueur des zones de concentration
h. Hauteur des zones de concentration

**Revendications**

1. Procédé de concentration d'un milieu (1) contenant des particules micrométriques dispersées dans un fluide (3), par acoustophorèse, **caractérisé en ce qu'**il comprend :

   - une circulation en continu et suivant un flux laminaire, du milieu (1) à concentrer successivement au travers de n zones (5) de concentration,
   - une application, dans chaque zone (5) de concentration, d'un champ acoustique stationnaire de fréquence de résonance propre sur le flux du milieu (1) à concentrer de sorte à agglomérer les particules sous forme d'une pluralité de bandes (2), et
   - une récupération d'un premier sous-milieu comprenant les particules sous forme de bandes (2) à la sortie des n zones (5) de concentration par un peigne muni d'une pluralité de dents (13), chaque dent comprenant un canal longitudinal récupérant une bande de particules, les dents (13) étant espacées par un espace inter-dents (14) au travers desquels circule un deuxième sous-milieu correspondant au milieu entre les bandes ,

   dans lequel la fréquence de résonance du champ acoustique émis dans la nième zone (5a) de concentration est supérieure à la fréquence de résonance du champ acoustique émis dans la nième + 1 zone (5b) de concentration de sorte à agglomérer successivement les particules sous forme de bandes (2) de plus en plus espacées.

2. Procédé suivant la revendication 1, dans lequel la circulation est paramétrée de sorte à ce que le milieu (1) circule suivant un flux ayant un débit compris entre 1 et 200 L/h.

3. Procédé suivant l'une quelconque des revendications précédentes dans lequel la fréquence de résonance du champ acoustique émis dans la nième zone (5a) de concentration est supérieure d'un ordre de grandeur à la fréquence de résonance du champ acoustique émis dans la nième+1 zone (5b) de concentration.

4. Procédé suivant l'une quelconque des revendications précédentes dans lequel au moins un champ acoustique est orthogonal à la direction de circulation du milieu.

5. Procédé suivant l'une quelconque des revendications précédentes dans lequel les particules sont des micro-algues.

6. Procédé suivant l'une quelconque des revendications précédentes dans lequel les particules ont une taille inférieure à $100\mu$m, préférentiellement inférieure ou égale à $80\mu$m, plus préférentiellement comprise entre $5\mu$m inclus et $80\mu$m inclus.

7. Procédé suivant l'une quelconque des revendications précédentes dans lequel la fréquence de résonance du champ acoustique émis dans la nième zone (5a) de concentration est comprise entre 1 et 10 MHz, de préférence égale à 2MHz, et la fréquence de résonance du champ acoustique émis dans la nième+ 1 zone (5b) de concentration est comprise entre 150 et 400 kHz, de préférence égale à 250kHz.

8. Procédé selon l'une quelconque des revendications dans lequel les zones (5) de concentration sont configurées pour que les particules sous forme de bandes (2) soient de plus en plus larges.

9. Dispositif destiné à la concentration d'un milieu (1), circulant en continu, contenant des particules micrométriques dispersées dans un fluide (3), par acoustophorèse, **caractérisé en ce qu'**il comprend suivant un sens de circulation (12) du milieu (1):

   - une section d'entrée (4) destinée à recevoir le milieu (1) à concentrer et configurée pour générer un flux laminaire continu dudit milieu (1),
   - n zones (5) de concentration successives, chaque zone (5) de concentration comprenant une source ultrasonore configurée pour générer un champ acoustique stationnaire de fréquence de résonance propre, la fréquence de résonance d'une zone n (5a) étant supérieure à la fréquence de résonance d'une zone n+1 (5b) de sorte à agglomérer successivement les particules sous forme de bandes (2) de plus en plus espacées,

- une section de récupération (6) comprenant un moyen de récupération configuré pour récupérer un premier sous-milieu comprenant les particules agglomérées sous forme de bandes (2),

la section de récupération comprenant un peigne (7) muni d'une pluralité de dents (13), chaque dent comprenant un canal longitudinal destiné à récupérer une bande de particules, les dents (13) étant espacées par un espace inter-dents (14) au travers desquels circule un deuxième sous-milieu correspondant au milieu entre les bandes.

10. Dispositif selon la revendication précédente dans lequel chaque source acoustique des zones (5) de concentration comprend une première plaque piézoélectrique émettrice placée sur une paroi latérale (11) d'une zone (5) de concentration et une deuxième plaque piézoélectrique réflectrice placée parallèle à la première sur une paroi opposée de ladite zone (5) de concentration de sorte à générer une onde acoustique stationnaire orthogonale au sens de circulation (12) du milieu (1).

11. Dispositif selon l'une quelconque des deux revendications précédentes dans lequel la largeur (l) des n zones (5) de concentrations est configurée pour permettre la formation de plusieurs bandes (2) de particules agglomérées parallèles les unes aux autres avec largeur (l) = nombre de bandes (2) x ½ longueur d'onde.

12. Dispositif selon l'une quelconque des trois revendications précédentes dans lequel la largeur des n zones de concentration est identique de sorte que les bandes soient de plus en plus larges.

13. Dispositif selon l'une quelconque des quatre revendications précédentes dans lequel la longueur des n zones (5) de concentration est configurée pour que le temps de séjour soit supérieur au temps de migration des particules.

14. Dispositif selon l'une quelconque des cinq revendications précédentes dans lequel les n zones (5) de concentration successives sont directement connectées les unes à la suite des aux autres.

15. Dispositif selon l'une quelconque des six revendications précédentes dans lequel le peigne (7) est agencé de sorte que la direction longitudinale des canaux soit parallèle à la direction longitudinale des bandes (2) de particules.

16. Dispositif selon l'une quelconque des sept revendications précédentes dans lequel le nombre de dents (13) est égal au nombre de bandes (2) dans la zone (5) de concentration précédant directement la section de récupération (6).

FIG. 1

FIG. 2

Europäisches
Patentamt

European
Patent Office

Office européen
des brevets

**RAPPORT DE RECHERCHE EUROPEENNE**

Numéro de la demande

EP 17 20 2179

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2006/037915 A1 (STRAND DAVID [US] ET AL) 23 février 2006 (2006-02-23) | 1-9, 11-14 | INV. A61M1/36 |
| Y | * alinéa [0072] - alinéa [0075]; revendication 1; figures 3,4,20 * * alinéas [0001], [0004] * | 15,16 | B01D43/00 B01D21/28 B01J19/10 C12M1/00 |
| | ----- | | |
| X | US 2011/123392 A1 (DIONNE JASON [US] ET AL) 26 mai 2011 (2011-05-26) | 9-14 | |
| Y | * alinéa [0009]; revendications 1,4,10; figures 2,3,4 * * alinéa [0015] - alinéa [0016] * * alinéas [0042], [0053] * | 15,16 | |
| | ----- | | |
| Y | EP 0 147 032 A1 (UNILEVER PLC [GB]; UNILEVER NV [NL]) 3 juillet 1985 (1985-07-03) * figure 8 * | 15,16 | |
| | ----- | | |
| A | US 2016/279540 A1 (PRESZ JR WALTER M [US] ET AL) 29 septembre 2016 (2016-09-29) * revendications 1-20; figures 2, 3 * | 1-16 | |
| | ----- | | **DOMAINES TECHNIQUES RECHERCHES (IPC)** |
| A | US 2010/078384 A1 (YANG TAHUA [US]) 1 avril 2010 (2010-04-01) * alinéa [0077]; figure 17 * | 1 | A61M B01D B01J C12M |
| | ----- | | |

Le présent rapport a été établi pour toutes les revendications

| Lieu de la recherche | Date d'achèvement de la recherche | Examinateur |
|---|---|---|
| Munich | 23 mars 2018 | García Alonso, Nuria |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

    & : membre de la même famille, document correspondant

EPO FORM 1503 03.82 (P04C02)

**EP 3 323 444 A1**

## ANNEXE AU RAPPORT DE RECHERCHE EUROPEENNE
## RELATIF A LA DEMANDE DE BREVET EUROPEEN NO.

EP 17 20 2179

23-03-2018

| Document brevet cité au rapport de recherche | | Date de publication | Membre(s) de la famille de brevet(s) | | Date de publication |
|---|---|---|---|---|---|
| US 2006037915 | A1 | 23-02-2006 | US WO | 2006037915 A1 03102737 A2 | 23-02-2006 11-12-2003 |
| US 2011123392 | A1 | 26-05-2011 | US US US | 2011123392 A1 2014202876 A1 2016347628 A1 | 26-05-2011 24-07-2014 01-12-2016 |
| EP 0147032 | A1 | 03-07-1985 | DE EP JP JP US WO | 3481281 D1 0147032 A1 H0679682 B2 S61500278 A 4743361 A 8501892 A1 | 15-03-1990 03-07-1985 12-10-1994 20-02-1986 10-05-1988 09-05-1985 |
| US 2016279540 | A1 | 29-09-2016 | CN US WO WO | 107635634 A 2016279540 A1 2016154475 A1 2017165014 A1 | 26-01-2018 29-09-2016 29-09-2016 28-09-2017 |
| US 2010078384 | A1 | 01-04-2010 | CA EP JP JP US WO | 2737654 A1 2352570 A2 5642684 B2 2012503776 A 2010078384 A1 2010036667 A2 | 01-04-2010 10-08-2011 17-12-2014 09-02-2012 01-04-2010 01-04-2010 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010036667 A

**Littérature non-brevet citée dans la description**

- **KAYANI et al.** Optofluidics incorporating actively controlled micro- and nano-particles. *BIOMICRO-FLUIDICS,* 2012, vol. 6, 031501